(19) Europäisches Patentamt
European Patent Office
Office européen des brevets

(11) **EP 4 445 831 A1**

(12) **EUROPEAN PATENT APPLICATION**

(43) Date of publication:
**16.10.2024  Bulletin 2024/42**

(21) Application number: **23167695.8**

(22) Date of filing: **13.04.2023**

(51) International Patent Classification (IPC):
**A61B 5/00** (2006.01)

(52) Cooperative Patent Classification (CPC):
**A61B 5/0066; A61B 5/0073; A61B 5/4288;
A61B 5/4312**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **PAULUSSEN, Elvira Johanna Maria
Eindhoven (NL)**

• **DAMODARAN, Mathivanan
Eindhoven (NL)**
• **JOHNSON, Mark Thomas
Eindhoven (NL)**
• **PRESURA, Cristian Nicolae
Eindhoven (NL)**
• **VERKRUIJSSE, Willem
Eindhoven (NL)**

(74) Representative: **Philips Intellectual Property &
Standards
High Tech Campus 52
5656 AG Eindhoven (NL)**

(54) **A MONITORING SYSTEM AND METHOD FOR MONITORING MILK FLOW DURING BREASTFEEDING OR MILK EXPRESSION**

(57)    There is provided a system for estimating milk flow during breastfeeding or milk expression. This can be achieved by using a Doppler OCT imaging apparatus to generate images of a breast, and a processor to determine a milk flow velocity and cross-sectional area of a milk duct in order to ultimately estimate a milk flow value. Proposed concepts thus aim to utilize the depth penetration imaging capabilities of a Doppler OCT imaging apparatus in order to provide a small system able to monitor milk flow in real-time, and potentially be integrated into a nipple shield, breast pump, or brassiere type garment.

FIG. 1

## Description

FIELD OF THE INVENTION

This invention relates to a monitoring system and method for monitoring milk flow during breastfeeding or during milk expression, in particular for determining milk flow quantity.

BACKGROUND OF THE INVENTION

Breastfeeding is the most natural way of infant feeding and is associated with many short term and long term medical and neurodevelopmental advantages. It is well known that breastfeeding drastically reduces the risk of sudden infant death syndrome, respiratory tract infections, gastrointestinal tract infections, necrotizing enterocolitis, allergic diseases, inflammatory bowel disease, celiac disease, obesity, childhood leukemia and lymphomia. It also improves neurodevelopmental outcomes. Consequently, the American Academy of Pediatrics strongly recommends mothers to breastfeed for 1 year or longer.

Besides the clear health benefits, breastfeeding also improves maternal-infant bonding. It is therefore most natural that women want to breast feed. However, breastfeeding is also associated with a variety of problems. Some mothers are not able to breast feed, breastfeeding can be painful, and mothers find it often challenging to assess whether their baby is provided with sufficient milk.

Mothers often have doubts about whether they produce and give sufficient milk to their babies. In addition, mastitis, cracked nipples and sore nipples are significantly associated with poor positioning and attachment, which are crucial for effective breastfeeding. As a result, many mothers unwillingly quit early with breastfeeding.

In a breast, multiple milk ducts are present, and each of them originates from a specific region containing the milk secreting alveoli.

To provide greater insights into the milk flow of the lactating human breast, different technological approaches have been developed. In a clinical setting, ultrasound imaging and ultrasound Doppler technology are promising tools to investigate milk flow or to detect a pathological lactating breast. It has for example been proposed in US 2009/0054771 to use Doppler ultrasound sensors, integrated into a wearable brassiere, to monitor milk flow during breastfeeding sessions. Alternatively, it has been proposed to monitor physiological changes in the mammary cutaneous perfusion during milk ejection using Laser Doppler perfusion monitoring. Overall milk flow volumes may also be measured using a nipple shield with an integrated flow sensor.

SUMMARY OF THE INVENTION

The invention is defined by the claims.

According to examples in accordance with an aspect of the invention, there is provided a system for estimating milk flow during breastfeeding or milk expression.

The system comprises: a Doppler optical coherence tomography, OCT, imaging apparatus adapted to generate one or more Doppler OCT images of a breast during the expression of milk from the breast; and a processor adapted to: determine a milk flow velocity and a cross-sectional area of a milk duct in the breast from the one or more Doppler OCT images; and estimate a milk flow value based on the determined milk flow velocity and the determined cross-sectional area.

Proposed concepts thus aim to provide schemes, solutions, concepts, designs, methods and system pertaining to estimating milk flow during breastfeeding or milk expression. In particular, embodiments aim to provide a system for estimating milk flow during breastfeeding or milk expression by using Doppler optical coherence tomography to determine a velocity of a milk flow and a cross-sectional area of a milk duct, and using this information, to estimate a milk flow. In this way, the amount of milk that is being expressed and/or that the baby is consuming may be tracked in real-time, thereby providing reassurance and comfort to parents. If milk flow is found to be inadequate, for example, the parents may then be informed so that they can make adjustments.

In other words, it is proposed that by using Doppler OCT images, the velocity of a milk flow and the geometry of a milk duct may be determined, and from this information, a milk flow value can be estimated. Doppler OCT images provide a way to give a 1-dimensional (A-scan), 2-dimensional (B-scan) or 3-dimensional (C-scan) representation of optical back scattering or back reflection in a cross-sectional plane or volume of tissue. This information can then subsequently be processed to estimate a milk flow value, such as, total milk volume expressed. Doppler OCT is an extension of traditional OCT, whereby the Doppler effect principle is utilized to achieve high resolution tomographic images of moving structures, e.g. blood cells. In this way, the velocity of objects, such as milk particles, can be determined.

In OCT, many one-dimensional depth scans (A-scans) may be performed at several locations to create a two-dimensional image (B-scan) or volumetric 3D images (C-scan). Milk flow velocity, direction, and volume in the milk ducts may then be determined by analyzing the Doppler signal of these repeated scans of milk ducts. From these determined parameters, further values may be estimated such as a milk flow value, or potentially an indication of milk duct clogging.

By performing Doppler OCT imaging at the right wavelength, for example, in the range of 700-1300nm, image penetration depth of 2 to 3mm can be achieved in most tissues, such as a breast. At these wavelengths, noise signals of tissue closer to the skin surface are also inherently suppressed. Milk ducts under the surface of the nipple, along with the milk flowing within them, may then be measured and studied using Doppler OCT imaging. Moreover, by determining a milk flow velocity and combining it with a determined cross-sectional area of the milk ducts, an absolute milk flow value may then be estimated. For example, the milk flow value may be the absolute milk flow rate or total milk volume expressed.

Doppler OCT imaging can be performed at home using a small system that could, for example, fit into a nipple shield. In this way, a system may be provided that can be easily integrated into the busy workflow of a mom. The imaging and processing may also be performed in real-time so that the parents may be informed of how much milk the mother is expressing or the baby is consuming in real-time. This can therefore provide reassurance to the parents and increase the efficiency of the milk-expressing or breastfeeding workflow.

In summary, using a Doppler OCT system, real-time measurements of milk flow within milk ducts at depth becomes possible. From Doppler OCT images, milk flow velocity may be determined as well as the cross-sectional area of the milk ducts, and by combining this information, a milk flow value may be estimated. The milk flow value may be milk volumetric flow within the ducts or total milk volume expressed.

Ultimately, an improved system for estimating milk flow during breastfeeding or milk expression may be supported by the proposed concept(s).

In some embodiments, the Doppler OCT imaging apparatus may comprise: a light source adapted to emit, towards the breast, a first light, wherein the first light is at least partially coherent and of an infrared wavelength; and at least one detector configured to: determine a first interference signal between backscattered light from the breast tissue and a reference light; and generate an OCT image from the first interference signal.

Traditional OCT, also known as LOCT, requires light with partial coherence to work, and the optimum coherence length is determined by the desired imaging depth. If the coherence length is too short, then the desired imaging depth cannot be reached. If the coherence length is too long, however, disturbance from other surfaces or tissue depths may occur. Fully coherent light, i.e. laser light, may be used however, if using SLD-based and Swept Source laser based OCTs.

In some embodiments, the Doppler OCT imaging apparatus may be configured to generate a plurality of velocity measurements over time, and wherein the processor may be adapted to determine a likelihood of a blockage in the milk duct based on one or more of: a stepped reduction of milk flow velocity; a rate of increase in milk flow velocity, wherein the rate of increase falls below a first threshold; and a rate of decrease in milk flow velocity, wherein the rate of decrease falls below a second threshold.

It has been realized that the measurements of milk flow velocities, as well as estimating milk flow values, can also be utilized to provide an early indication of blockage of milk ducts. Therefore, in addition to quantitative measurement of the milk flow, further measurements can be derived that may provide useful early indications of milk duct clogging. For example, a step like reduction of milk flow rate in a longitudinal measurement following a standard sucking event may indicate blocking. In addition, a slower rate of flow onset (and a slower rate of flow decrease) after the sucking event, may again indicate a blockage has developed (e.g. measure of the time to maximum flow/time for flow to return to zero, slope of the flow onset/decrease).

In some embodiments, the Doppler OCT imaging apparatus may be adapted to generate a 3-dimensional OCT image. A 3-dimensional OCT image is commonly referred to as a "C-scan". By performing C-scans, 3D Doppler data is provided within the 3D scan data. Therefore, knowledge of the Doppler angle of the flow is not required for deriving absolute flow.

In some embodiments, the wavelength of light used by the Doppler OCT imaging apparatus may be between 700 and 1700nm, preferably between 750 and 1300 nm. This range of wavelengths has been determined to result in an effective penetration depth in breast tissue. Accordingly, accurate information can be derived.

In some embodiments, the Doppler OCT imaging apparatus may be further adapted for balanced detection and comprises at least two light detectors. Balanced detection enables noise in the images to be reduced but requires at least two light detectors to work, one for measuring the desired signal and the other for directly measuring the source of the emitted light. This allows the cancelling out of any noise due to the source noise. There are a number of different suitable ways of implementing balanced detection in this invention.

In some embodiments, the Doppler OCT imaging apparatus may be further adapted for polarization imaging. The system may be further enhanced by employing Doppler OCT enhancement crossed-polarization method, where polarization optics are used to decrease background surface reflections and increase signal to noise ratio (SNR) of the OCT images. There are a number of different suitable ways of implementing polarized imaging in this invention.

In some embodiments, the Doppler OCT imaging apparatus may comprise a self-mixing interferometry detector. Self-mixing interferometry, SMI, is an effect where the light emitted by a laser re-enters the laser cavity after being scattered by an object and interferes with the light present in the laser cavity. SMI hardware is especially suited to measure relative velocities, and laser sensors making use of SMI are commercially available, such as the Philips laser beetle™.

In some embodiments, a light source of the Doppler OCT imaging apparatus comprises a swept-laser source, or comprises a broadband source and a spectrometer. Both of these embodiments are suitable for a high-resolution, high-speed Fourier domain (FD) OCT system, thereby keeping measurement times short and avoiding large motion artifacts while imaging ducts in a moving nipple with a high enough resolution.

In some embodiments, the processor may be adapted to generate a map of milk flow levels for one or more regions of the breast. Generating a map of milk flow for different regions may be useful for diagnosing particular problem areas or blockages. By creating a milk flow map, milk ducts with low flow can be detected early before severe problems develop, because the reduction in milk flow from a localized area of the breast becomes visible. The image may for example colour code different flow levels. The generated flow map allows personalized guidance to be given on how to position the baby during a next feeding session, reducing the occurrence of blocked milk ducts and discomfort. Thresholds may be applied to the flow levels to enable detection of blocked ducts.

According to another aspect of the invention, there is provided a brassiere type garment, a nipple shield, or a breast pump expression kit comprising any herein disclosed system.

Integrating the flow sensor arrangement into a brassiere type garment, nipple shield, or a breast pump expression kit provides highly convenient and unobtrusive operation. Accordingly, milk quantity may be determined with no additional steps or systems. The system may also be miniaturized by (on-chip) photonic integration to meet form factor requirements.

According to another aspect of the invention, there is provided a method for estimating milk flow during breastfeeding or milk expression. The method comprises: generating one or more Doppler optical coherence tomography, OCT, images of a breast during the expression of milk from the breast; determining a milk flow velocity and a cross-sectional area of a milk duct in the breast from the one or more Doppler OCT images; and estimating a milk flow value based on the determined milk flow velocity and the determined cross-sectional area.

In some embodiments, generating one or more Doppler OCT images of a breast during the expression of milk from the breast may further comprise: emitting, towards the breast, a first light, wherein the first light is at least partially coherent and of an infrared wavelength; determining a first interference signal between backscattered light from the breast tissue and a reference light; and generating an OCT image from the first interference signal.

In some embodiments, the method may further comprise the step of determining one or more of: a stepped reduction of milk flow velocity; a rate of increase in milk flow velocity, wherein the rate of increases falls below a first threshold; and a rate of decrease in milk flow velocity, wherein the rate of decrease falls below a second threshold, to determine a likelihood of a blockage in the milk duct.

According to another aspect of the invention, there is provided a computer program comprising computer program code means for implementing the method of any herein disclosed method when said program is run on a processing system.

Thus, there may be proposed concepts for estimating milk flow during breastfeeding or milk expression, and this may be done via Doppler OCT imaging.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 is a block diagram of a system for estimating milk flow during breastfeeding or milk expression according to a proposed embodiment;

Fig. 2 is a diagram illustrating how volumetric milk flow through a milk duct may be determined;

Figs. 3A-3D are block diagrams of systems for estimating milk flow during breastfeeding or milk expression according to various proposed embodiments respectively;

Fig. 4 is a simplified block diagram of a nipple shield comprising a system for estimating milk flow during breastfeeding or milk expression according to a proposed embodiment;

Fig. 5 is a simplified diagram of a nipple shield on a breast comprising a system for estimating milk flow during breastfeeding or milk expression according to a proposed embodiment;

Fig. 6 is a simplified flow diagram of a method for estimating milk flow during breastfeeding or milk expression according to a proposed embodiment;

Fig. 7 is a more in-depth flow diagram of a method for estimating milk flow during breastfeeding or milk expression according to a proposed embodiment; and

Fig. 8 illustrates an example of a computer within which one or more parts of an embodiment may be employed.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

Implementations in accordance with the present disclosure relate to various techniques, methods, schemes and/or solutions pertaining to estimating milk flow during breastfeeding or milk expression. According to proposed concepts, a number of possible solutions may be implemented separately or jointly. That is, although these possible solutions may be described below separately, two or more of these possible solutions may be implemented in one combination or another.

Embodiments of the invention aim to provide a system for estimating milk flow during breastfeeding or milk expression. This can be achieved by using a Doppler OCT imaging apparatus to generate images of a breast, and a processor to determine a milk flow velocity and cross-sectional area of a milk duct in order to ultimately estimate a milk flow value.

Proposed concepts thus aim to utilize the depth penetration imaging capabilities of a Doppler OCT imaging apparatus in order to provide a small system able to monitor milk flow in real-time. This small system may then be integrated into a nipple shield, breast pump, or brassiere type garment.

In other words, using a Doppler OCT system, real-time measurements of milk flow within milk ducts at depth becomes possible. From Doppler OCT images, milk flow velocity may be determined as well as the cross-sectional area of the milk ducts, and by combining this information, a milk flow value may be estimated such as milk volumetric flow within the ducts or total milk volume expressed.

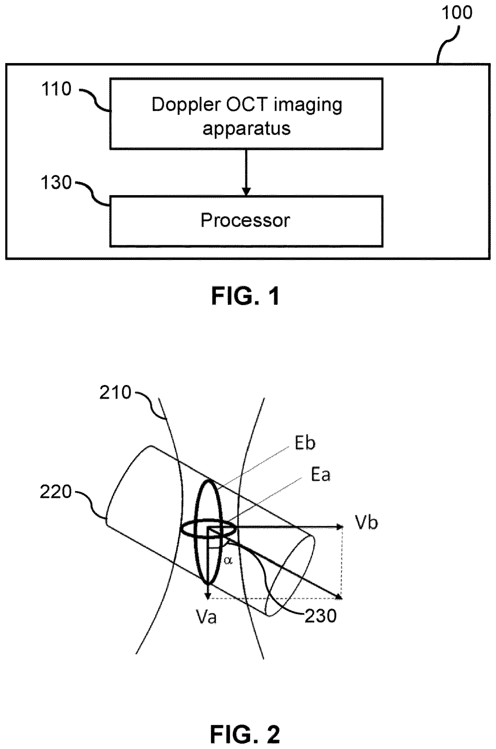

Referring now to Fig. 1, there is depicted a block diagram of a system 100 for estimating milk flow during breastfeeding or milk expression according to a proposed embodiment.

The system 100 comprises a Doppler optical coherence tomography, OCT, imaging apparatus 110 adapted to generate one or more Doppler OCT images of a breast during expression of milk from the breast. The system 100 further comprises a processor 130 adapted to determine a milk flow velocity and a cross-sectional area of a milk duct in the breast from the one or more Doppler OCT images, and estimate a milk flow value based on the determined milk flow velocity and the determined cross-sectional area.

In other words, from the generated one or more Doppler OCT images, the velocity of milk flow may be determined via the Doppler signal, and a cross-sectional area of one or more milk ducts may be directly determined from the OCT image scans: (axial) A-, (lateral) B-scans, and optionally (lateral) C-scans.

In this embodiment, the Doppler OCT imaging apparatus 110 is adapted to generate a 3-dimensional OCT image. A 3-dimensional OCT image is commonly referred to as a "C-scan". By performing C-scans, 3D Doppler data is provided within the 3D scan data. Therefore, knowledge of the Doppler angle of the flow is not required for deriving absolute flow. In other embodiments, however, the Doppler OCT imaging apparatus may be adapted to only generate 2-dimensional OCT images (B-scan) or 1-dimensional OCT images (A-scan). In these cases, the processor 130 will need to analyze the images in order to determine a Doppler angle of the milk flow.

Cross-sectional imaging of the milk ducts by B-scans can be achieved by laterally combining a series of A-scans. To achieve good axial resolution and to avoid motion artifacts, scans should be taken at a high-speed, i.e. A-scan rate should be substantially higher than pumping rate. As milk ducts stretch and deform due to the pumping motion during breastfeeding or milk expression, A-, B-, and C-scans should ideally be performed at a turning point, e.g. when the nipple is most stretched. A motion or proximity sensor may be included in the system 100 for triggering the OCT scans. Ideally, in embodiments in which the system 100 is integrated into a breast pump, the moment of scanning should be synced to the pumping frequency of the breast pump.

In this embodiment, the wavelength of light used by the Doppler OCT imaging apparatus 110 is between 750 and 1300 nm. This range of wavelengths has been determined to result in an effective penetration depth in breast tissue. Accordingly, accurate information can be derived. Any wavelength of light between 700 and 1700nm may be suitable, however.

In a study by DT Ramsay et al.[1], the depth of the main collecting branch of ducts at the base of the nipple for left and right breasts were 4.50 ± 1.98mm and 4.74 ± 1.59mm respectively. Milk ducts are superficial, easily compressible and echoes within the duct represent fat globules in breastmilk. The low number and size of the ducts, the rapid branching under the areola and the absence of sinuses suggest that ducts transport breastmilk, rather than store it. By performing OCT imaging using light at a wavelength at 1300nm, for example, image penetration depth of 2 to 3mm can be achieved in most tissues. It has been demonstrated in studies[2] that scattering coefficients of bulk skin and adipose tissue are around 20% lower at 1.7 $\mu$m compared to 1.3 $\mu$m. There is a peak of effective imaging penetration using light with a wavelength of 1300nm, and a slightly lower peak at 1700nm. It has also been found that imaging in the areola/nipple area where duct concentration (duct volume fraction) is high with respect to the intraglandular tissue that penetration depth is higher than in typical tissue.

[1] RAMSAY DT et al., Anatomy of the lactating human breast redefined with ultrasound imaging. Journal of Anatomy. June 2005, 206(6), pages 525-534.

[2] SHARMA U et al., Long-wavelength optical coherence tomography at 1.7 microns for enhanced imaging depth. Optics Express. 2008, 16(24), pages 19712-19723.

In some embodiments, the Doppler OCT imaging apparatus 110 may be configured to generate a plurality of velocity measurements over time, and the processor 130 may be adapted to determine a likelihood of a blockage in the milk duct based on one or more of: a stepped reduction of milk flow velocity; a rate of increase in milk flow velocity, wherein the rate of increase falls below a first threshold; and a rate of decrease in milk flow velocity, wherein the rate of decrease falls below a second threshold.

It has been realized that the measurements of milk flow velocities, as well as estimating milk flow values, can also be utilized to provide an early indication of blockage of milk ducts. Therefore, in addition to quantitative measurement of the milk flow, further measurements can be derived that may provide useful early indications of milk duct clogging. For example, a step like reduction of milk flow rate in a longitudinal measurement following a standard sucking event may indicate blocking. In addition, a slower rate of flow onset (and a slower rate of flow decrease) after the sucking event, may again indicate a blockage has developed (e.g. measure of the time to maximum flow/time for flow to return to zero, slope of the flow onset/decrease).

The use of thresholds provides an efficient way to determine whether the milk duct is clogged or at risk of clogging, either partially, substantially, or fully. The thresholds may be predetermined based on generalized data, or may be personalized to each user based on past measurements. This personalization may be performed automatically by the processor 130.

In some embodiments, the processor 130 may also be adapted to generate a map of milk flow levels for one or more regions of the breast. Generating a map of milk flow for different regions may be useful for diagnosing particular problem-areas or blockages. By creating a milk flow map, milk ducts with low flow can be detected early before severe problems develop, because the reduction in milk flow from a localized area of the breast becomes visible. The image may for example colour code different flow levels. The generated flow map allows personalized guidance to be given on how to position the baby during a next feeding session, reducing the occurrence of blocked milk ducts and discomfort. Thresholds may be applied to the flow levels to enable detection of blocked ducts.

In some embodiments, the processor 130 may be configured to alert the user if a volume or flow of milk that the baby is receiving falls below a threshold value. The processor 130 may also be configured to provide guidance on breast feeding based on the estimated milk flow value. The processor 130 may also be configured to translate the milk flow value to a drinking or sucking rate.

In some embodiments, the system 100 may further be configured to measure a user's health vital signs via their skin. Example vital signs that could be measured are vitamin levels, stress levels, nutrition status, etc.

Referring now to Fig. 2, there is depicted a diagram showing how volumetric milk flow through a milk duct 220 may be determined.

To calculate absolute milk flow velocities, and since Doppler OCT can only measure the velocity component parallel to the optical beam, both the Doppler angle 230 (the angle between flow direction and optical beam) and the structure of the duct 220 (elliptical axes Ea and Eb) needs to be determined by performing A-scans 210 and B-scans. In this case, the actual flow velocity can be calculated by taking the vector sum of the Doppler flow of the A-scan 210 (Va) and the B-scan (Vb).

The average velocity, v, in a milk duct 220 can be defined as the distance, d, the milk in a duct travels in a period of time, t. Flow rate, Q, can be defined as the volume of fluid passing by a location, P, through an area, A, during a period of time, t. Hence, in simple terms, the flow rate can be calculated by finding the average flow velocity through the Doppler signal combined with estimating the cross-sectional area of the milk duct 220 using the OCT intensity images. By doing repeated and adjacent OCT B-scans, the flow velocity in the direction of a probe beam, $v_z$, can be calculated. The absolute velocity, $v_{abs}$, can then be calculated by estimating the angle between the probe beam 210 and the duct 220.

In more detail, taking $\lambda_c$ as the center wavelength of the source light, $\Delta f$ as the Doppler shift, $\alpha$ as the Doppler angle 230, and n as the tissue refractive index, the milk flow velocity, v, can be given by:

$$v = \frac{\lambda_c \cdot \Delta f}{2n \cdot cos\alpha} \quad\quad\quad (1)$$

Then using the milk flow velocity, v, calculated using equation (1), the flow volume rate, Q, can be calculated using:

$$Q = v \cdot CA \quad\quad\quad (2)$$

CA stands for the cross-sectional area of the duct 220, which can be determined from an OCT image. In other words, taking Ea as the horizontal elliptical axis of the milk duct 220 and Eb as the vertical elliptical axis, the flow volume rate, Q, can be given by:

$$Q = (\frac{\lambda_c \cdot \Delta f}{2n \cdot cos\alpha}) \cdot Ea \cdot Eb \quad\quad\quad (3)$$

The total milk volume, V, expressed by the milk duct over a certain time period dt (milk sampling interval dt) can then be given by:

$$V = \int Q \, dt \qu\quad\quad\quad (4)$$

It should be noted that by performing C-scans, 3D Doppler data is collected and therefore knowledge of the Doppler angle 230 is not required for deriving absolute flow. This may be referred to as an en-face plane-based flow method, and in this method, total flow can be derived from the cross-sectional area in a plane bisecting ducts perpendicular to the optical beam. For instance, the measured velocity vector milk flow and the normal vector of the cross-sectional area may be parallel. In order to cover multiple ducts, multiple systems with optical beams passing the ducts can be arranged in a rotational symmetric set-up around the areola area. In this way, duct blockages may be detected. Longitudinal data could be collected and used to follow the lactation process, i.e. monitor a decreased volume of milk expressed or decreased breastfeeding. This information may then be used to guide the mother to phase out breastfeeding in a gentle and gradual way.

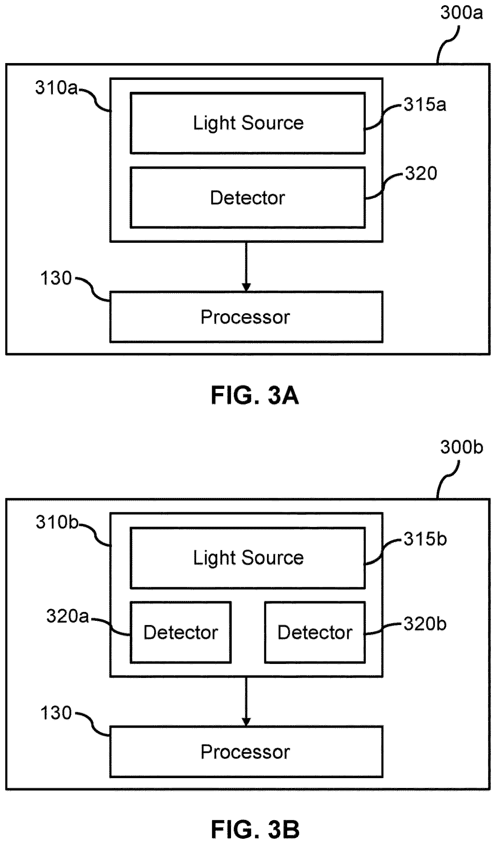

Referring now to Fig. 3A, there is depicted a block diagrams of a system 300a for estimating milk flow during breastfeeding or milk expression according to a proposed embodiment.

The processor 130 is the same as was described in relation to Fig. 1, however, in this embodiment the Doppler OCT imaging apparatus 310a comprises a light source 315a and a detector 320. The light source 315a is adapted to emit, towards the breast, a first light, wherein the first light is at least partially coherent and of an infrared wavelength. Traditional OCT, also known as LOCT, requires light with partial coherence to work, and the optimum coherence length is determined by the desired imaging depth. If the coherence length is too short, then the desired imaging depth cannot be reached. If the coherence length is too long, however, disturbance from other surfaces or tissue depths may occur. Fully coherent light, i.e. laser light, may be used however, if using SLD-based and Swept Source laser based OCTs.

The detector 320 is configured to determine a first interference signal between backscattered light from the breast tissue and a reference light. A reference light may be produced by light from the light source 315a being reflected back towards the detector 320 by a mirror. Lenses may be placed throughout the system 300a in order to focus or disperse the light where required. The detector 320 then generates a Doppler OCT image from the first interference signal.

Referring now to Fig. 3B, there is depicted a block diagram of a system 300b for estimating milk flow during breastfeeding or milk expression according to a proposed embodiment. The processor 130 is the same as was described in relation to Fig. 1, however, in this embodiment the Doppler OCT imaging apparatus 310b comprises a light source 315b, which may be substantially the same as light source 315a, and two detectors 320a and 320b. The use of at least two light detectors, 320a and 320b, allows the imaging apparatus 320b to be adapted for balanced detection. In another embodiment, there may be a plurality of light detectors. Balanced detection enables noise in the OCT images to be reduced but requires at least two light detectors to work, comparing split signals coming from the reference, the sample, and the source directly. This allows the cancelling out of any noise due to the source noise. There are a number of different suitable ways for implementing balanced detection in this invention.

As an example, one suitable specific implementation of balanced detection in this invention may include the Doppler OCT imaging apparatus 310b further comprising an optical circulator to redirect light from the light source 315a towards a reference and measurement branch, and one first photodiode for cancelling out laser noise for image analysis.

The apparatus 310b may further include a splitter, such as a 50/50 splitter, to split the light that comes from the reference and measurement branch into a second photodiode and a third photodiode. The processor 130 may then compare the light from the light source 315a and the light from the reference and measurement branch in the first photodiode (or second and third photodiode) in order to reduce noise and improve signal-to-noise ratio. This implementation may reduce laser intensity noise, but other implementations of balanced detection are possible and suitable for the present invention. It should be noted that for RF balancing of system noise, more hardware in the system 310b (or any other herein disclosed embodiment of the system) may be needed such as beam splitters and polarization controllers.

In some embodiments, the Doppler OCT imaging apparatus 310b may be further adapted for polarization imaging. The system 300b may be further enhanced by employing Doppler OCT enhancement crossed-polarization method, where polarization optics are used to decrease background surface reflections and increase signal to noise ratio (SNR) of the OCT images. There are a number of different suitable ways of implementing polarized imaging in this invention, and polarized imaging may be implemented into any herein disclosed embodiment of the system.

Generally, there are two types of polarization sensitive OCT systems. One using polarization controllers with polarizing beam splitters, PBS, and one not using polarization controllers. The first requires more complex signals processing, due to the required balancing between P and S state of polarization in the polarization controllers. Polarization controller optics may be photonics integrated. The latter type, without a polarization controller, would be the more preferred embodiment in the present invention due to compactness and cost considerations. These types of polarization sensitive OCT systems use quarter wave plates, QWPs, instead of a polarization controller, and there are generally two types of implementations. In the first type, a reference branch in the OCT system is split up into two branches using two reference mirrors. The first branch has QWPs and the second branch does not.

In the second type of implementation of a polarization sensitive OCT system without polarization controllers, there is provided one reference branch with a first QWP and one measurement branch with a second QWP. The two QWPs are aligned at 45 degrees with respect to one another, so that the two states of polarization in each branch are orthogonal, i.e. cross-polarized. Signal processing then consists of gathering co- and cross-polarized images to be separated into individual images. In this way, sample reflectivity, i.e. layered constructions of biological tissue, can be extracted from the images.

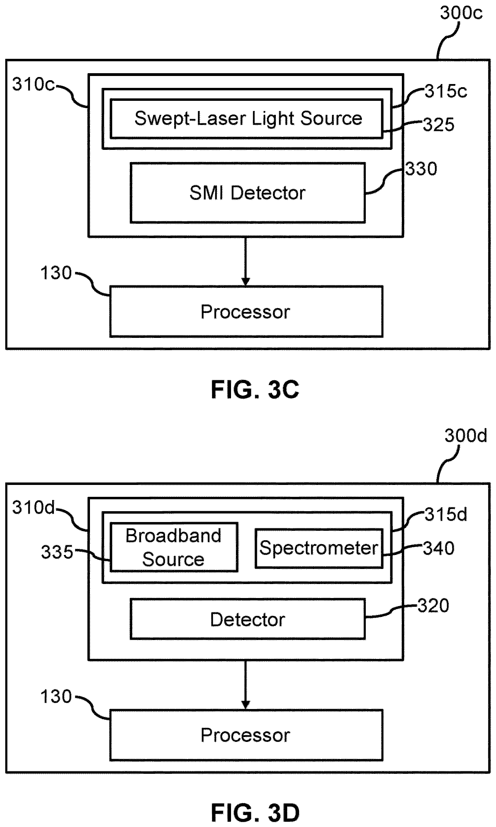

Referring now to Fig. 3C, there is depicted a block diagram of a system 300c for estimating milk flow during breastfeeding or milk expression according to a proposed embodiment. The processor 130 is the same as was described in relation to Fig. 1, however, in this embodiment the Doppler OCT imaging apparatus 310c comprises a self-mixing interferometry, SMI, detector 330 and the light source 315c comprises a swept-laser light source 325.

Self-mixing interferometry, SMI, is an effect where the light emitted by a laser re-enters the laser cavity after being scattered by an object and interferes with the light present in the laser cavity. SMI hardware, such as the SMI detector 330, is especially suited to measure relative velocities, and laser sensors making use of SMI are commercially available, such as the Philips laser beetle™.

The use of a swept-laser light source 325 allows for a high-resolution, high-speed Fourier domain (FD) OCT system 310c, thereby keeping measurement times short and avoiding large motion artifacts while imaging milk ducts in a moving nipple with high enough resolution.

For the utilisation of SMI and the SMI detector 330, however, the light source 315c need not be a swept-laser light source 325. The light source may instead be a super luminescent diode, SLD.

There are several advantages to using a super-luminescent laser diode in a system utilising SMI. First, it is possible to measure very small spatial regions (of the order of the coherence length of the SLD, usually in the tens of micrometres) irrespective of where the light that re-enters the laser cavity comes from. This is because only light coming from these regions will be coherent with the light inside the laser. Light coming from other spatial regions that re-enter the laser cavity will not be coherent with the light inside the cavity and thus they will create no interference signal. Secondly, it is possible to measure the velocity of different objects within a certain coherence region and disregard other regions. These coherence regions are referred to as ghost regions or autocorrelation peaks, and they are generally situated 1mm apart. These regions are located at precise distances with respect to the sensor (integer multiples of the length of the optical cavity). The light re-entering the optical cavity from other regions is essentially invisible for the sensor since it does not produce interference patterns.

In embodiments which include an SLD as a light source, the laser may be moved to probe different locations. This may comprise moving the laser within the system to scan the breast while the system is fixed on the breast, or it may comprise moving the whole system with respect to the breast, for instance, rotating or re-sitting the system. Alternatively, the breast could be moved with respect to the system until, for instance, a feedback signal is provided that informs the user that the position is good. Moving the laser within the system would be the preferred embodiment as this requires less user intervention. Moving the laser to probe different locations would be particularly useful to find the places where the milk flow gives the best signal. Since SLD SMI has the potential to discriminate among different regions, scanning in a Doppler OCT set-up may provide information about the best locations to measure milk flow.

Referring now to Fig. 3D, there is depicted a block diagram of a system 300d for estimating milk flow during breastfeeding or milk expression according to a proposed embodiment. The processor 130 is the same as was described in relation to Fig. 1, however, in this embodiment the Doppler OCT imaging apparatus 310d comprises a light source 315d which comprises a broadband source 335 and a spectrometer 340. The detector 320 may be the same as the detector as was described in relation to Fig. 3A.

The use of a broadband source 335 in conjunction with a spectrometer 340 allows for a high-resolution, high-speed Fourier domain (FD) OCT system 310d, thereby keeping measurement times short and avoiding large motion artifacts while imaging milk ducts in a moving nipple with high enough resolution.

In some embodiments, the spectrometer 340 may be replaced by a grating with a line sensor or a camera.

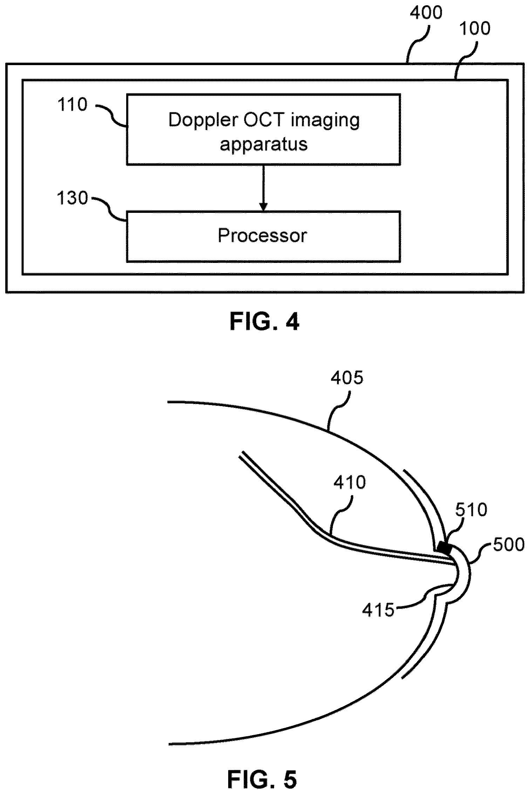

Referring now to Fig. 4, there is depicted a simplified block diagram of a nipple shield 400 comprising a system 100 for estimating milk flow during breastfeeding or milk expression according to a proposed embodiment. The system 100 is not limited to the system 100 of Fig. 1, but may be a system according to any of the herein disclosed embodiments. Furthermore, the nipple shield 400 may be replaced with a brassiere type garment or a breast pump expression kit, either electric or manual. Integrating the flow sensor arrangement into a brassiere type garment, nipple shield, or a breast pump expression kit provides highly convenient and unobtrusive operation. Ideally, the OCT apparatus should make contact with the nipple area or areola for greater effectiveness, however, this is not essential. The OCT apparatus could, for instance, still function hovering above the nipple area or areola. Accordingly, milk quantity may be determined with no additional steps or systems. The system may also be miniaturized by (on-chip) photonic integration to meet form factor requirements. Photonic integration contributes to the miniaturization and integration of optical systems.

The nipple shield 400 may also be replaced by a bottle, storage cup, breast pad, or thermal pad.

Referring now to Fig. 5, there is depicted a simplified diagram of a nipple shield 500 on a breast 405 comprising a system 510 for estimating milk flow during breastfeeding or milk expression according to a proposed embodiment. When integrated into a nipple shield 500 or a brassiere type garment or a breast pump expression kit, the system 510 should be placed so that in use the OCT apparatus is located over an area where the milk ducts 410 are close to the skin. The system 510 may then typically be placed within the nipple shield 500 so that in use they are close to the nipple 415.

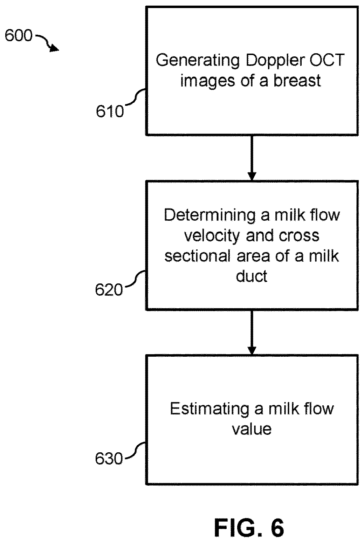

Referring now to Fig. 6, there is depicted a simplified flow diagram of a method 600 for estimating milk flow during breastfeeding or milk expression according to a proposed embodiment.

In step 610, one or more Doppler OCT images of a breast during the expression of milk from the breast are generated. In step 620, a milk flow velocity and a cross-sectional area of a milk duct in the breast from the one or more Doppler OCT images are determined. In step 630, a milk flow value based on the determined milk flow velocity and the determined cross-sectional area is estimated.

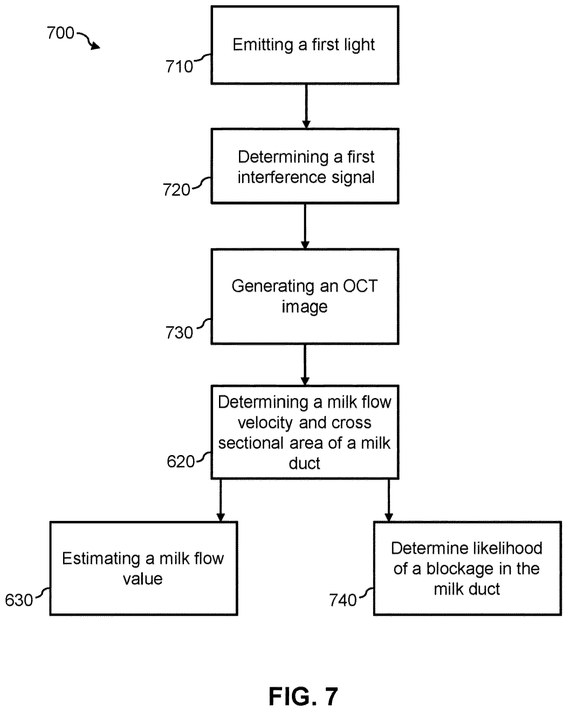

Referring now to Fig. 7, there is depicted a more in-depth flow diagram of a method 700 for estimating milk flow during breastfeeding or milk expression according to a proposed embodiment. In step 710, a first light is emitted towards the breast, wherein the first light is at least partially coherent and of an infrared wavelength. In step 720, a first interference signal is determined between backscattered light from the breast tissue and a reference light. In step 730, an OCT image is generated from the first interference signal. Steps 620 and 630 are the same as were described in relation to Fig. 6.

In step 740, one or more of the following are determined to determine a likelihood of a blockage in the milk duct: a stepped reduction of milk flow velocity; a rate of increase in milk flow velocity, wherein the rate of increases falls below a first threshold; and a rate of decrease in milk flow velocity, wherein the rate of decrease falls below a second threshold.

It should be noted that any herein disclosed processing does not need to be in real time. Images can for example be stored and analyzed after the breast pumping or breast feeding event, and the output may be given a short time afterwards. However, real time processing is also possible.

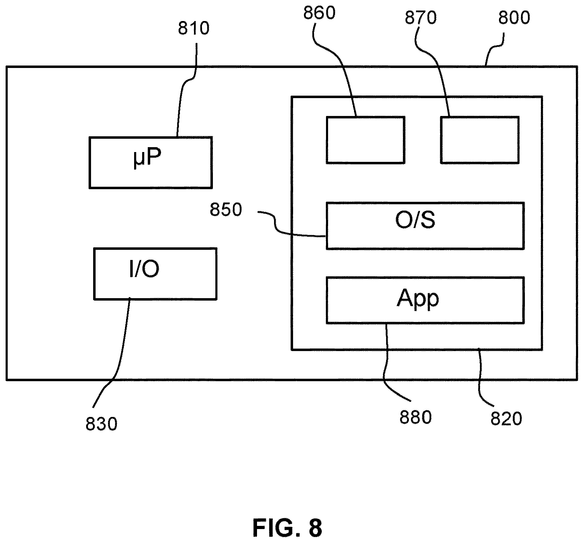

Fig. 8 illustrates an example of a computer 800 within which one or more parts of an embodiment may be employed. Various operations discussed above may utilize the capabilities of the computer 800. In this regard, it is to be understood that system functional blocks can run on a single computer or may be distributed over several computers and locations (e.g. connected via internet).

The computer 800 includes, but is not limited to, PCs, workstations, laptops, PDAs, palm devices, servers, storages, and the like. Generally, in terms of hardware architecture, the computer 800 may include one or more processors 810, memory 820 and one or more I/O devices 830 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 810 is a hardware device for executing software that can be stored in the memory 820. The processor 810 can be virtually any custom made or commercially available processor, a central processing unit (CPU), a digital signal processor (DSP), or an auxiliary processor among several processors associated with the computer 800, and the processor 810 may be a semiconductor based microprocessor (in the form of a microchip) or a microprocessor.

The memory 820 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 820 may incorporate electronic, magnetic, optical, and/or other types of storage media. Note that the memory 820 can have a distributed architecture, where various components are situated remote from one another, but can be accessed by the processor 810.

The software in the memory 820 may include one or more separate programs, each of which comprises an ordered listing of executable instructions for implementing logical functions. The software in the memory 820 includes a suitable operating system (O/S) 850, compiler 860, source code 870, and one or more applications 880 in accordance with exemplary embodiments. As illustrated, the application 880 comprises numerous functional components for implementing the features and operations of the exemplary embodiments. The application 880 of the computer 800 may represent various applications, computational units, logic, functional units, processes, operations, virtual entities, and/or modules in accordance with exemplary embodiments, but the application 880 is not meant to be a limitation.

The operating system 850 controls the execution of other computer programs, and provides scheduling, input-output control, file and data management, memory management, and communication control and related services. It is contemplated by the inventors that the application 880 for implementing exemplary embodiments may be applicable on all commercially available operating systems.

Application 880 may be a source program, executable program (object code), script, or any other entity comprising a set of instructions to be performed. When a source program, then the program is usually translated via a compiler (such as the compiler 860), assembler, interpreter, or the like, which may or may not be included within the memory 820, so as to operate properly in connection with the O/S 850. Furthermore, the application 880 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 830 may include input devices such as, for example but not limited to, a mouse, keyboard, scanner, microphone, camera, etc. Furthermore, the I/O devices 830 may also include output devices, for example but not limited to a printer, display, etc. Finally, the I/O devices 830 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 830 also include components for communicating over various networks, such as the Internet or intranet.

If the computer 800 is a PC, workstation, intelligent device or the like, the software in the memory 820 may further include a basic input output system (BIOS) (omitted for simplicity). The BIOS is a set of essential software routines that initialize and test hardware at start-up, start the O/S 850, and support the transfer of data among the hardware devices. The BIOS is stored in some type of read-only-memory, such as ROM, PROM, EPROM, EEPROM or the like, so that the BIOS can be executed when the computer 800 is activated.

When the computer 800 is in operation, the processor 810 is configured to execute software stored within the memory 820, to communicate data to and from the memory 820, and to generally control operations of the computer 800 pursuant to the software. The application 880 and the O/S 850 are read, in whole or in part, by the processor 810, perhaps buffered within the processor 810, and then executed.

When the application 880 is implemented in software it should be noted that the application 880 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 880 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 6 and 7, and the system of Figs. 1, 3A-3D and 4, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Figs. 1, 3A-3D and 4 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

**Claims**

1. A system (100) for estimating milk flow during breastfeeding or milk expression, comprising:

   a Doppler optical coherence tomography, OCT, imaging apparatus (110) adapted to generate one or more Doppler OCT images of a breast during the expression of milk from the breast; and
   a processor (130) adapted to:

   determine a milk flow velocity and a cross-sectional area of a milk duct in the breast from the one or more Doppler OCT images; and

estimate a milk flow value based on the determined milk flow velocity and the determined cross-sectional area.

2.  The system of claim 1, wherein the Doppler OCT imaging apparatus comprises:

    a light source adapted to emit, towards the breast, a first light, wherein the first light is at least partially coherent and of an infrared wavelength; and
    at least one detector configured to:

    determine a first interference signal between backscattered light from the breast tissue and a reference light; and
    generate an OCT image from the first interference signal.

3.  The system of any preceding claim, wherein the Doppler OCT imaging apparatus is configured to generate a plurality of velocity measurements over time, and wherein the processor is adapted to determine a likelihood of a blockage in the milk duct based on one or more of:

    a stepped reduction of milk flow velocity;
    a rate of increase in milk flow velocity, wherein the rate of increase falls below a first threshold; and
    a rate of decrease in milk flow velocity, wherein the rate of decrease falls below a second threshold.

4.  The system of any preceding claim, wherein the Doppler OCT imaging apparatus is adapted to generate a 3-dimensional OCT image.

5.  The system of any preceding claim, wherein the wavelength of light used by the Doppler OCT imaging apparatus is between 700 and 1700nm, preferably between 750 and 1300 nm.

6.  The system of any preceding claim, wherein the Doppler OCT imaging apparatus is further adapted for balanced detection and comprises at least two light detectors.

7.  The system of any preceding claim, wherein the Doppler OCT imaging apparatus is further adapted for polarization imaging.

8.  The system of any preceding claim wherein the Doppler OCT imaging apparatus comprises a self-mixing interferometry detector.

9.  The system of any preceding claim, wherein a light source of the Doppler OCT imaging apparatus comprises a swept-laser source, or comprises a broadband source and a spectrometer.

10. The system of any preceding claim, wherein the processor is adapted to generate a map of milk flow levels for one or more regions of the breast.

11. A brassiere type garment, nipple shield, or a breast pump expression kit comprising the system of any preceding claim.

12. A method (600) for estimating milk flow during breastfeeding or milk expression comprising the steps of:

    generating (610) one or more Doppler optical coherence tomography, OCT, images of a breast during the expression of milk from the breast;
    determining (620) a milk flow velocity and a cross-sectional area of a milk duct in the breast from the one or more Doppler OCT images; and
    estimating (630) a milk flow value based on the determined milk flow velocity and the determined cross-sectional area.

13. The method of claim 12, wherein generating one or more Doppler OCT images of a breast during the expression of milk from the breast comprises:

    emitting, towards the breast, a first light, wherein the first light is at least partially coherent and of an infrared wavelength;

determining a first interference signal between backscattered light from the breast tissue and a reference light; and

generating an OCT image from the first interference signal.

14. The method of any of claims 12 or 13, comprising the step of determining one or more of:

a stepped reduction of milk flow velocity;

a rate of increase in milk flow velocity, wherein the rate of increases falls below a first threshold; and

a rate of decrease in milk flow velocity, wherein the rate of decrease falls below a second threshold,

to determine a likelihood of a blockage in the milk duct.

15. A computer program comprising computer program code means which is adapted, when said program is run on a computer, to implement the method claims 12 to 14.

100

110

Doppler OCT imaging
apparatus

130

Processor

**FIG. 1**

210

Eb

Ea

220

Vb

α

Va

230

**FIG. 2**

**FIG. 3A**

**FIG. 3B**

**FIG. 3C**

**FIG. 3D**

400
100

110

Doppler OCT imaging apparatus

130

Processor

## FIG. 4

405

410

510

500

415

## FIG. 5

600

Generating Doppler OCT
images of a breast

610

Determining a milk flow
velocity and cross
sectional area of a milk
duct

620

Estimating a milk flow
value

630

**FIG. 6**

700 ⟍

| 710 | Emitting a first light |

↓

| 720 | Determining a first interference signal |

↓

| 730 | Generating an OCT image |

↓

| 620 | Determining a milk flow velocity and cross sectional area of a milk duct |

↓      ↓

| 630 | Estimating a milk flow value |

| 740 | Determine likelihood of a blockage in the milk duct |

# FIG. 7

**FIG. 8**

# EUROPEAN SEARCH REPORT

Europäisches
Patentamt
European
Patent Office
Office européen
des brevets

Application Number

EP 23 16 7695

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 3 827 737 A1 (KONINKLIJKE PHILIPS NV [NL]) 2 June 2021 (2021-06-02) * paragraphs [0001], [0014] – [0022], [0035], [0040] – [0047], [0050], [0056], [0074], [0075], [0079] – [0083] * * figure 2a * | 1-15 | INV. A61B5/00 |
| Y | US 2016/120524 A1 (SUEHIRA NOBUHITO [JP]) 5 May 2016 (2016-05-05) * paragraphs [0050], [0059], [0069], [0079] – [0083], [0089] * | 1-15 | |
| A | US 2021/169398 A1 (SORGINI FRANCESCA [IE] ET AL) 10 June 2021 (2021-06-10) * the whole document * | 1-15 | |

TECHNICAL FIELDS
SEARCHED      (IPC)

A61B

The present search report has been drawn up for all claims

| Place of search | Date of completion of the search | Examiner |
|---|---|---|
| The Hague | 20 June 2023 | Kowalczyk, Szczepan |

EPO FORM 1503 03.82 (P04C01)

## ANNEX TO THE EUROPEAN SEARCH REPORT
## ON EUROPEAN PATENT APPLICATION NO.

EP 23 16 7695

This annex lists the patent family members relating to the patent documents cited in the above-mentioned European search report.
The members are as contained in the European Patent Office EDP file on
The European Patent Office is in no way liable for these particulars which are merely given for the purpose of information.

20-06-2023

| Patent document cited in search report | | Publication date | Patent family member(s) | | Publication date |
|---|---|---|---|---|---|
| EP 3827737 | A1 | 02-06-2021 | AU | 2020393248 A1 | 14-07-2022 |
| | | | CA | 3162590 A1 | 03-06-2021 |
| | | | CN | 114765945 A | 19-07-2022 |
| | | | EP | 3827737 A1 | 02-06-2021 |
| | | | EP | 4064960 A1 | 05-10-2022 |
| | | | US | 2022395218 A1 | 15-12-2022 |
| | | | WO | 2021104858 A1 | 03-06-2021 |
| US 2016120524 | A1 | 05-05-2016 | JP | 6711880 B2 | 17-06-2020 |
| | | | JP | 2015016300 A | 29-01-2015 |
| | | | JP | 2019013788 A | 31-01-2019 |
| | | | US | 2016120524 A1 | 05-05-2016 |
| | | | WO | 2014199641 A2 | 18-12-2014 |
| US 2021169398 | A1 | 10-06-2021 | CN | 112512410 A | 16-03-2021 |
| | | | EP | 3809952 A1 | 28-04-2021 |
| | | | GB | 2576028 A | 05-02-2020 |
| | | | JP | 2021533357 A | 02-12-2021 |
| | | | US | 2021169398 A1 | 10-06-2021 |
| | | | WO | 2020025337 A1 | 06-02-2020 |

EPO FORM P0459

For more details about this annex : see Official Journal of the European Patent Office, No. 12/82

## REFERENCES CITED IN THE DESCRIPTION

**Patent documents cited in the description**

• US 20090054771 A

**Non-patent literature cited in the description**

• **RAMSAY DT et al.** Anatomy of the lactating human breast redefined with ultrasound imaging. *Journal of Anatomy,* June 2005, vol. 206 (6), 525-534

• **SHARMA U et al.** Long-wavelength optical coherence tomography at 1.7 microns for enhanced imaging depth. *Optics Express,* 2008, vol. 16 (24), 19712-19723